(19) Europäisches Patentamt
European Patent Office
Office européen des brevets

(11) EP 4 618 088 A1

(12) EUROPEAN PATENT APPLICATION
published in accordance with Art. 153(4) EPC

(43) Date of publication:
17.09.2025 Bulletin 2025/38

(51) International Patent Classification (IPC):
G16B 5/00 (2019.01)    G16B 20/20 (2019.01)
G16B 40/00 (2019.01)

(21) Application number: 23889112.1

(22) Date of filing: 07.11.2023

(52) Cooperative Patent Classification (CPC):
G16B 5/00; G16B 20/20; G16B 40/00

(86) International application number:
PCT/KR2023/017782

(87) International publication number:
WO 2024/101854 (16.05.2024 Gazette 2024/20)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 08.11.2022 KR 20220148029

(71) Applicants:
• Invites Genomics Co., Ltd.
Jeju-si, Jeju-do 63243 (KR)
• CG Invites Co., Ltd.
Seoul 07802 (KR)

(72) Inventors:
• CHO, Yun Sung
Seongnam-si Gyeonggi-do 13539 (KR)
• JUN, Je Hoon
Suwon-si Gyeonggi-do 16239 (KR)
• LEE, Hwang Yeol
Yongin-si Gyeonggi-do 16953 (KR)
• CHUNG, Ok Sung
Hwaseong-si Gyeonggi-do 18499 (KR)

(74) Representative: Zacco Sweden AB
P.O. Box 5581
Löjtnantsgatan 21
114 85 Stockholm (SE)

(54) METHOD FOR PREDICTING IMMUNOGENIC EPITOPE AND APPARATUS USING SAME

(57) A method for predicting the immunogenicity of an epitope, comprising: calculating the degree to which each of factors involved in a biological process for the epitope of a tumor cell and properties of the epitope affects the immunogenicity of the epitope; performing at least one of standardization and normalization on the calculated value; and inputting a value in which at least one of the standardization and normalization has been performed into a pre-trained artificial intelligence model to predict the immunogenicity of the epitope, wherein the biological process comprises an antigen processing stage, an antigen presentation stage, an immunogenicity stage, and a tumor microenvironment, and the factors involved in the tumor microenvironment are at least one of inflammatory response, B-cell linear epitope, and B-cell conformational epitope.

FIG. 4

## Description

### Technical Field

The present disclosure relates to a method for predicting an immunogenic epitope, and a device using the same, and more particularly, to a method for predicting an immunogenic epitope by integrating various factors involved in many biological processes and epitope properties, and a device using the same.

### Background Art

Epitope recognition by T cells plays a key role in an immune response to pathogens and tumors. Neoantigens expressed by cancer mutations may activate immune cells in the body and contribute to treat cancer. Neoantigens may solve the problem of low responsiveness of immuno-oncology drugs by converting a cold tumor, which is not recognized by T cells, into a hot tumor, which is highly penetrable by T cells. That is, neoantigens may be mutationally expressed to be cancer cell-specific, allowing T cells that recognize the neoantigen/neoepitope to selectively proliferate and/or attack only tumor cells.

The generation of immunogenic epitopes may involve factors of biological processes related to an immune response, such as MHC class I/II binding affinity, proteasomal cleavage, TAP transporter efficiency, peptide-MHC (pMHC) stability, and T-cell receptor (TCR)-epitope interactions. In addition, properties of the epitope peptide sequence, for example, post-translational modification, sequence similarity to known epitope, dissimilarity to self-proteome, degree of functional change of mutation, antiinflammatory/pro-inflammatory properties of the epitope, and tumor microenvironment (TME) may also affect the immunogenicity of an epitope.

The various factors involved in various biological processes, such as MHC binding affinity, proteasomal cleavage, TAP transporter efficiency, peptide-MHC (pMHC) stability, and T-cell receptor (TCR) interactions, and the properties of epitope sequences, have been studied to predict epitopes that induce immune responses in silico. For example, a tool called PRIME predicts epitopes that induce an immune response by integrating a factor of MHC I binding affinity in an antigen presentation stage and a factor of TCR recognition in an immunogenicity stage of the biological process, and a tool called MHCflurry 2.0 predicts epitopes that induce an immune responses by integrating a factor involved in an antigen processing stage and a factor of MHC I binding affinity in an antigen presentation stage of the biological process. In the recently published research by the TESLA consortium, criteria/parameters for predicting an immunogenic epitope, considering MHC I binding affinity, peptide-MHC (pMHC) binding stability, agretopicity (a ratio of mutant binding affinity to wild-type binding affinity), foreignness (homology to known pathogenic peptides), etc., were also presented.

### Summary of Invention

### Technical Problem

Up to now, methods have been developed to predict epitopes that induce an immune response by individually utilizing various factors involved in many biological processes and/or epitope properties, or by combining a small number of factors and/or properties. However, a plurality of factors and/or properties may contribute together or sequentially to determining the immunogenicity of an epitope. The present disclosure additionally proposes various factors (biomarkers) that can be considered to accurately predict the immunogenicity of an epitope, and proposes a method for integrating and analyzing the factors and/or properties involved in/acting on immunogenicity, and a device using the same.

### Solution to Problem

According to some aspects of the disclosure, a method for predicting the immunogenicity of an epitope, comprises: calculating the degree to which each of factors involved in a biological process for the epitope of a tumor cell and properties of the epitope affects the immunogenicity of the epitope, performing at least one of standardization and normalization on the calculated value, and inputting a value in which at least one of the standardization and normalization has been performed into a pre-trained artificial intelligence model to predict the immunogenicity of the epitope, wherein the biological process comprises an antigen processing stage, an antigen presentation stage, an immunogenicity stage, and a tumor microenvironment, and the factors involved in the tumor microenvironment are at least one of inflammatory response, B-cell linear epitope, and B-cell conformational epitope.

According to some aspects, the calculating of the degree to which each of the factors involved in the biological process for the epitope and the properties of the epitope affects the immunogenicity of the epitope comprises calculating the degree using an algorithm, a program, or a tool.

According to some aspects, the properties of the epitope are at least one of functional impact by mutations, phosphorylation, hydrophobicity, similarity to known epitopes, dissimilarity to self (human reference)-proteome, and peptide stability.

According to some aspects, the factors involved in the antigen processing stage of the biological process are at least one of proteasomal cleavage and TAP transporter efficiency.

According to some aspects, the factors involved in the antigen presentation stage of the biological process are at least one of MHC I binding affinity, pMHC stability, average binding affinity to MHC I alleles, cross-reactivity to MHC I alleles, average binding affinity to MHC II alleles, and cross-reactivity to MHC II alleles.

According to some aspects, the average binding affinity to MHC I/MHC II alleles is calculated as in [Equation 3]:

[Equation 3]

$$\sum_{i}^{n} Fi \cdot Bi$$

where n is the type/number of MHC I/MHC II alleles, F is the frequency of occurrence in all or a specific number of the corresponding MHC I/MHC II alleles, and B is the binding affinity of the epitope to the corresponding MHC I/MHC II alleles, wherein the cross-reactivity to MHC I/MHC II alleles is a value obtained by collecting MHC I/MHC II alleles occurring in a population, calculating the binding affinity to all or a specific number of MHC I/MHC II alleles for the epitope to be calculated, and calculating a case where the binding affinity is less than a predetermined reference value to calculate how many MHC I/MHC II alleles the epitope is to react with.

According to some aspects of the disclosure, a computer-readable recording medium recording a computer program for executing the method of any one of claims 1 to 6.

According to some aspects of the disclosure, a device for predicting the immunogenicity of an epitope, comprises: an input/output device receiving an epitope or outputting a prediction result of the immunogenicity of the epitope; a storage device storing an artificial intelligence model trained to predict the immunogenicity of an epitope, based on at least some of factors involved in a biological process for an epitope of a tumor cell and properties of the epitope; and a computing device calculating the degree to which each of the factors involved in the biological process for the epitope of the tumor cell and the properties of the epitope affects the immunogenicity of the epitope, performing at least one of standardization and normalization on the calculated value, and inputting a value where at least one of the standardization and normalization has been performed into the trained artificial intelligence model to predict the immunogenicity of the epitope, wherein the biological process comprises an antigen processing stage, an antigen presentation stage, an immunogenicity stage, and a tumor microenvironment, and the factors involved in the tumor microenvironment are at least one of inflammatory response, B-cell linear epitope, and B-cell conformational epitope.

**Advantageous Effects**

According to the present disclosure, immunogenic epitopes can be predicted with high accuracy by simultaneously utilizing various factors involved in many biological processes that lead to the immunogenicity of an epitope and the properties of the epitope. The immunogenic epitopes predicted with high accuracy can be expected to have higher efficacy and lower side effects when developing a neoantigen-based vaccine.

**BRIEF DESCRIPTION OF THE DRAWINGS**

FIG. 1 illustrates an example of a biological process of a tumor cell.
FIG. 2 illustrates factors of many biological processes that may affect the immunogenicity of an epitope and the epitope properties.
FIG. 3 illustrates an example of a process for learning immunogenic epitopes using an artificial intelligence model.
FIG. 4 illustrates an example of a process for predicting immunogenicity epitopes using an artificial intelligence model.
FIG. 5 illustrates an example of a configuration diagram of a device for predicting immunogenic epitopes using an artificial intelligence model.

## DETAILED DESCRIPTION

The following embodiments are only for explaining the present disclosure more specifically, and it will be obvious to those skilled in the art that the scope of the present disclosure is not limited by these examples according to the gist of the present disclosure. It should be understood that all modifications, equivalents, or substitutes included in the spirit and scope of the technology described below are included.

In the terms used herein, expressions of the singular should be understood to include the plural unless the context clearly indicates otherwise. The term "includes" and the like should be understood to mean the presence of the described features, numbers, steps, operations, components, parts, or combinations thereof, and not to exclude the possibility of the presence or addition of one or more other features, numbers, steps, operations, components, parts, or combinations thereof. The term and/or includes a combination of a plurality of related recited items or any one of a plurality of related recited items.

In performing a method or method of operation, each process/step constituting the method may occur differently from the stated order unless the context clearly indicates a particular order. That is, each processes/step may occur in the same order as stated, may be performed substantially simultaneously, or may be performed in reverse order.

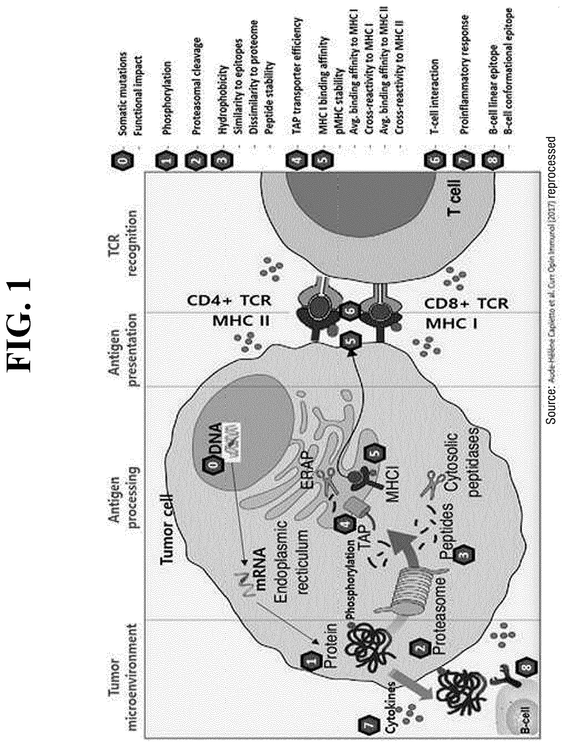

FIG. 1 illustrates an example of a biological process of a tumor cell.

Referring to FIG. 1, the biological process of a tumor cell may comprise an antigen processing stage, an antigen presentation stage, and an immunogenicity stage. A tumor microenvironment (TME) surrounding the tumor cell may also affect the biological process, and thus may be considered as a part of the biological process of the tumor cell. The biological process of the tumor cell is the same as or similar to the biological process of normal cells, but various factors may affect the generation and/or properties of epitopes at each stage.

The antigen processing stage is a stage in which DNA in the nucleus of a tumor cell is transcribed into mRNA and released into the cytoplasm, and the transcribed mRNA is transformed into a peptide and binds to MHC class I. The DNA of the tumor cell may be transformed due to mutations, etc. The mRNA may be translated and synthesized into a protein, and then fragmented into a peptide by the proteasome. The fragmented peptide is transported into an endoplasmic reticulum by a TAP transporter and binds to MHC class I.

The antigen presentation stage is a stage in which the peptide generated in the antigen processing stage binds to MHC class I and is presented on the cell surface as a peptide-MHC complex.

The immunogenicity stage is a stage in which the peptide-MHC complex presented on the cell surface is recognized by T cells.

The tumor microenvironment (TME) is an environment surrounding a cell, and the cell may be surrounded by blood vessels, immune cells, fibroblasts, bone marrow-derived inflammatory cells, lymphocytes, signaling molecules, and extracellular matrix. Tumor cells may affect the tumor microenvironment by inducing extracellular signal release, tumor cell angiogenesis promotion, and peripheral immune tolerance, and the tumor microenvironment may also affect the growth and/or metastasis of tumor cells. For example, if the disease that induces an immune response is cancer, the clonality of a sample may affect the immunogenicity of an epitope, and the distribution/proportion of immune cells, tumor infiltrating lymphocytes, RNA expression, etc. may also affect the immunogenicity of the generated epitope.

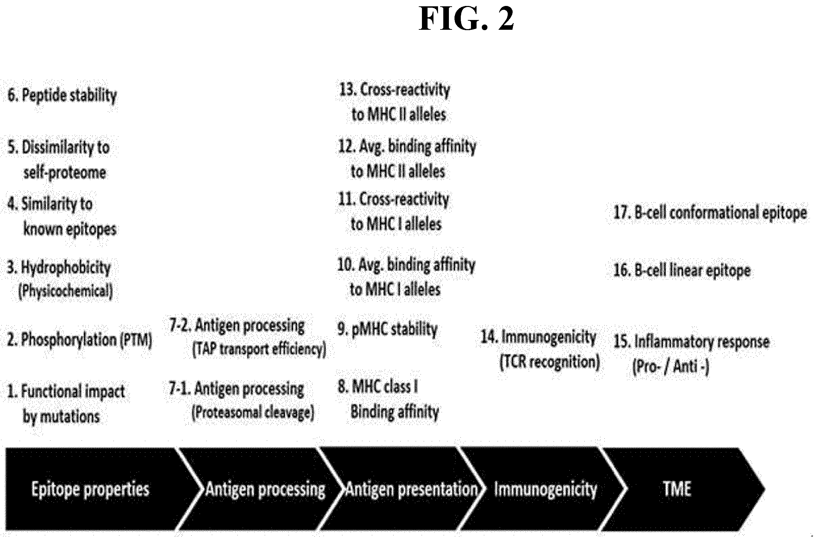

FIG. 2 illustrates factors of many biological processes that may affect the immunogenicity of an epitope and the epitope properties.

In addition to the biological processes described in FIG. 1, the immunogenicity of an epitope may also be affected by the properties of the epitope. For example, negative factors affecting the human body, such as toxicity and allergenicity of the epitope, may affect the immunogenicity of an epitope. The factors affecting the immunogenicity of an epitope may affect one at a time, but may also affect multiple factors simultaneously and/or sequentially.

Hereinafter, the epitope properties and the various factors that may affect the immunogenicity of an epitope in the biological process will be described in detail.

Among epitope properties, factors that may affect immunogenicity include functional impact by mutations, phosphorylation (one of PTM), hydrophobicity (or physicochemical hydrophobicity), similarity to known epitopes, dissimilarity to self (human reference)-proteome, peptide stability, etc.

Hereinafter, specific programs/algorithms/tools are described as examples to confirm the degree to which each factor affects the immunogenicity of an epitope, but are not limited thereto.

The functional impact by mutations is a factor that indicates the impact on the function of a protein due to somatic mutations that cause tumors. Epitopes (peptides) containing mutations with large functional impact are naturally eliminated by existing immune cells, but epitopes containing mutations with relatively small functional impact may exhibit immunogenicity at a higher frequency. Programs such as PROVEAN or PolyPhen-2 may be used to confirm the degree to which the functional impact by mutations affects the immunogenicity of an epitope.

Phosphorylation is a factor that indicates the degree to which the MHC class I ligand of an epitope for which immunogenicity is to be predicted is phosphorylated. A program such as NetMHCphosPan may be used to confirm the degree to which phosphorylation affects the immunogenicity of an epitope.

Hydrophobicity is a factor that indicates whether the epitope for which immunogenicity is to be predicted is hydrophobic. A program such as ProtFP may be used to confirm the degree to which hydrophobicity affects the immunogenicity of an epitope.

Similarity to known epitopes is a factor that indicates whether the epitopes for which immunogenicity is to be predicted are similar to epitopes known to be immunogenic (or non-immunogenic). A program such as blastP may be used to confirm the degree to which the similarity to known epitopes affects the immunogenicity of an epitope.

The dissimilarity to self (human reference)-proteome is a factor that indicates whether the epitope for which immunogenicity is to be predicted is dissimilar to a self-proteome. Here, the self-proteome may be a human-referenced self-proteome. For example, the pairwise2 module of Python Bio package may be used to confirm the degree to which dissimilarity to self (human reference)-proteome affects the immunogenicity of an epitope.

Epitope (peptide) stability is a factor that indicates the stability of the epitope (peptide) itself. Previously, the stability of peptide-MHC binding was used, but the stability of the epitope (peptide) itself was not used. If the stability of the epitope is low, the opportunity to bind/interact with the MHC molecule is reduced, so the possibility of having immunogenicity may be low. A program such as ProtParam may be used to confirm the degree to which epitope (peptide) stability affects the immunogenicity of an epitope.

In the antigen processing stage of the biological process, proteasomal cleavage and/or TAP transporter efficiency may be used to predict the immunogenicity of an epitope. A program such as NetCTLpan may be used to confirm the degree to which proteasomal cleavage and TAP transporter efficiency affect the immunogenicity of an epitope.

In the antigen presentation stage of the biological process, at least some of the MHC I binding affinity, pMHC stability, average binding affinity to MHC I alleles, cross-reactivity to MHC I alleles, average binding affinity to MHC II alleles, and cross-reactivity to MHC II alleles may be used to predict an epitope that elicits an immune response.

MHC class I binding affinity is a factor that indicates the degree to which an epitope for which immunogenicity is to be predicted binds to MHC class I. Programs such as NetMHCpan and/or MHCflurry may be used to confirm the degree to which MHC class I binding affinity affects the immunogenicity of an epitope.

pMHC stability is a factor that indicates the peptide-MHC stability of an epitope. A program such as NetMHCstabpan may be used to confirm the degree to which pMHC stability affects the immunogenicity of an epitope.

Traditionally, the binding affinity to an epitope-MHC pair has been utilized. However, epitopes and MHC may interact many to many. The average binding affinity to MHC I alleles is a factor that indicates the degree to which an epitope is recognized by multiple MHC I alleles by measuring the average binding size of a single epitope to multiple MHC I alleles. The cross-reactivity to MHC I alleles is a factor that indicates how many MHC I alleles may bind to the corresponding epitope by calculating the number of MHC I alleles that bind to a single epitope based on a specific reference binding affinity value.

The average binding affinity to MHC I alleles may be expressed as a value obtained by collecting MHC I alleles occurring in a population, calculating the binding affinity to all or a specific number of MHC I alleles for the epitope to be calculated, and calculating the frequency of alleles as a weight, as shown in [Equation 1]:

[Equation 1]

$$\sum_{i}^{n} Fi \cdot Bi$$

where n is the type/number of MHC I alleles, F is a value between 0 and 1, representing the frequency of occurrence in all or a specific number of the corresponding MHC I alleles, and B is the binding affinity of the epitope to the corresponding MHC I alleles.

The cross-reactivity to MHC I alleles may be expressed as a value obtained by collecting MHC I alleles occurring in a population, calculating the binding affinity to all or a specific number of MHC I alleles for the epitope to be calculated, and counting of a case where the binding affinity is less than a reference value (e.g., 50 nM) to calculate how many MHC I alleles the epitope is to react with.

The average binding affinity to MHC I alleles and the cross-reactivity to MHC I alleles for MHC I may also be applied to MHC II. Epitopes that bind to MHC I alleles that elicit a CD8+ (cytotoxic) T-cell response and also bind to MHC II alleles that elicit a CD4+ (helper) T-cell response may elicit a higher immune response. The average binding affinity to MHC II alleles is a factor that indicates the degree to which an epitope is recognized by multiple MHC II alleles by measuring the average binding size of a single epitope to multiple MHC II alleles. The cross-reactivity to MHC II alleles is a factor that indicates how many MHC II alleles may bind to the corresponding epitope by calculating the number of MHC II alleles that bind to a single epitope based on a specific reference binding affinity value.

The average binding affinity to MHC II alleles may be expressed as a value obtained by collecting the MHC II alleles occurring in a population, calculating the binding affinity to all or a specific number of MHC II alleles for the epitope to be calculated, and calculating an average using the frequency of alleles as a weight, as shown in [Equation 2]:

[Equation 2]

$$\sum_{i}^{n} Fi \cdot Bi$$

where n is the type/number of MHC II alleles, F is the frequency of occurrence in all or a specific number of the corresponding MHC II alleles, and B is the binding affinity of the epitope to the corresponding MHC II alleles.

The cross-reactivity to MHC II alleles may be expressed as a value obtained by collecting MHC II alleles occurring in a population, calculating the binding affinity to all or a specific number of MHC II alleles for the epitope to be calculated, and calculating a case where the binding affinity is less than a reference value (e.g., adjust consensus percentile rank 10%) to calculate how many MHC II alleles the epitope is to react with.

In the immunogenicity stage of the biological process, immunogenicity may be used to predict an epitope that elicits an immune response. At least one of programs such as IEDB, PRIME, DeepHLApan, and DeepImmuno may be used to confirm the degree to which immunogenicity affects the immunogenicity of an epitope.

In the tumor microenvironment, at least one of inflammatory response, B-cell linear epitope, and B-cell conformational epitope may be considered as a factor predicting an epitope that elicits an immune response.

Immune cells may regulate an immune response by secreting inflammatory substances such as cytokines. The ability of an epitope to induce these cytokines is calculated, and if the epitope induces cytokines excessively/insufficiently, the immune response may be suppressed. At least one of the programs such as PIP-EL, AIPpred, and IFNepitope may be used to confirm the degree to which the inflammatory response affects the immunogenicity of an epitope.

Somatic mutations that cause tumors may be recognized by B-cells. B-cells may recognize both linear epitopes in the form of peptides, and conformational epitopes in the form of protein structures. B-cell linear epitopes are factors that indicate linear epitope potential, and B-cell confirmational epitopes are factors that indicate conformational epitope potential. For example, the Bepipred program may be used to conform the degree to which B-cell linear epitopes affect the immunogenicity of an epitope. For example, Discotope may be used to confirm the degree to which B-cell conformational epitopes affect the immunogenicity of an epitope.

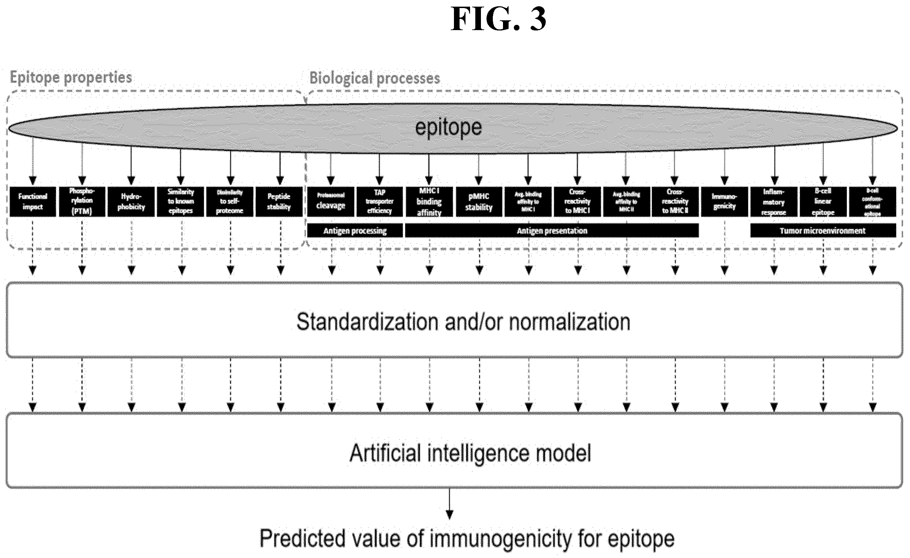

FIG. 3 illustrates an example of a process for learning immunogenic epitopes using an artificial intelligence model.

Referring to FIG. 3, an artificial intelligence model may be used to predict immunogenic epitopes. The artificial intelligence model may be various learning models, such as machine learning, neural networks, deep learning, logistic regression, AdaBoost, LogitBoost, XgBoost, Support vector machines, and random forest methods. In addition, an ensemble machine learning method (e.g., bagging, stacking, voting, boosting, etc.) that integrates multiple machine learning algorithms or statistical methods may also be utilized. The input to the artificial intelligence model may be at least some of the various factors that may affect the immunogenicity of an epitope described in FIG. 2, and the output may be a prediction result of whether the epitope is immunogenic. The various factors that may affect the immunogenicity of an epitope may be input into an artificial intelligence model as values calculated using a public program/algorithm/tool, or as values calculated using the method proposed in the present disclosure. According to an embodiment, the values derived using a program/algorithm/tool may have different value ranges or resolutions depending on each of factors or the applied program/algorithm/tool, and thus may be standardized or/and normalized and used.

Training of the artificial intelligence model may be performed using both immunogenic and non-immunogenic epitopes. The training of the artificial intelligence model may be performed by integrating multiple factors, or may be performed separately for factor. The data used to train the artificial intelligence model may be publicly available data stored in a database such as the Immune Epitope Database (IEDB).

According to an embodiment, immunogenic/non-immunogenic epitopes may be predicted for each factor. The results calculated for each factor may be evaluated based on statistical methods such as area under the curve (AUC), precision-recal AUC (prAUC), and positive predictive value (PPV), and the programs/algorithms/tool may be added or excluded, or factors themselves that affect the immunogenicity of the epitope may also be excluded. Here, the addition/subtraction of the program/algorithm/tool and/or individual factors may vary depending on the target disease. For example, different factors and/or combinations of programs/algorithms/tools may be formed depending on the type of cancer.

The result of the artificial intelligence model is a predicted value of immunogenicity for the epitope. The predicted value of immunogenicity for the epitope is a probability that the epitope is immunogenic or non-immunogenic, and may be a value between 0 and 1. For example, if the predicted value of immunogenicity for the epitope is close to 1, it may be judged as immunogenic, and if the predicted value of immunogenicity for the mutant epitope is close to 0, it may be judged as non-immunogenic.

According to another embodiment, the result of the artificial intelligence model may be a result of determining whether the epitope is immunogenic. The artificial intelligence model may compare the predicted value of immunogenicity for the epitope with a reference value and determine it as immunogenic or non-immunogenic. The reference value may vary depending on any one or a combination of the tumor type, purpose, and situation.

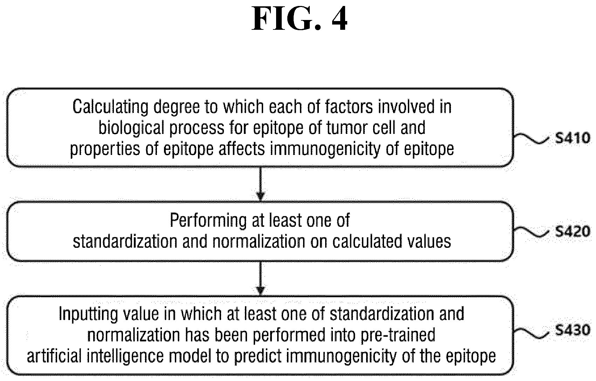

FIG. 4 illustrates an example of a process for predicting immunogenicity epitopes using an artificial intelligence model.

Referring to FIG. 4, a method of predicting the immunogenicity of an epitope according to an embodiment of the present disclosure may first include a process (S410) of calculating the degree to which each of factors involved in a biological process for the epitope of a tumor cell and properties of the epitope affects the immunogenicity of the epitope. The biological process may comprise an antigen processing stage, an antigen presentation stage, an immunogenicity stage, and a tumor microenvironment, wherein the factors involved in the tumor microenvironment may be at least one of inflammatory response, B-cell linear epitope, and B-cell conformational epitope.

A method of predicting the immunogenicity of an epitope may include a process (S420) of performing at least one of standardization and normalization on the calculated value. The calculated value may have different ranges and may also have different resolutions, so standardization and/or normalization may be required. If the calculated values have the same range or resolution that affects the immunogenicity of the epitope to be derived, standardization and normalization may not be required.

A method of predicting the immunogenicity of an epitope may include a process (S430) of inputting a value in which at least one of the standardization and normalization has been performed into a pre-trained artificial intelligence model to predict the immunogenicity of the epitope.

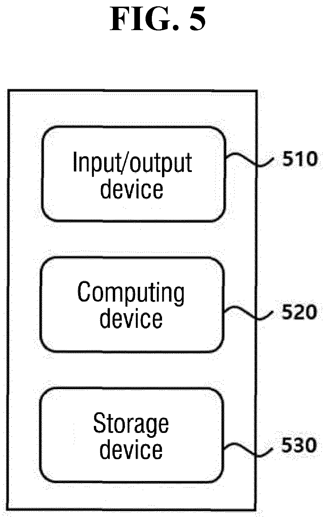

FIG. 5 illustrates an example of a configuration diagram of a device for predicting immunogenic epitopes using an artificial intelligence model.

Referring to FIG. 5, a device for predicting the immunogenic epitopes using an artificial intelligence model may include an input/output device 510, a storage device 520, and a computing device 530.

The input/output device 510 (or an input and output device) may be configured to receive an epitope or output a prediction result. The input/output device 510 may be configured to receive an epitope from a user or another device. Here, the input/output device 510 has been illustrated as one device, but may be configured as separate devices. For example, the input device may be a mouse and/or a keyboard, and the output device may be a display such as a monitor or a speaker.

The storage device 520 may store an artificial intelligence model trained to predict the immunogenicity of an epitope trained, based on at least some of the factors involved in a biological process for the epitope of a tumor cell, and the properties of the epitope. The storage device 520 may store a programs/algorithms/tool required for data processing in addition to the artificial intelligence model.

The computing device 530 may calculate the degree to which each of the factors involved in the biological process for the epitope of the tumor cell, and the properties of the epitope affects the immunogenicity of the epitope, may perform at least one of standardization and normalization on the calculated value, and may input a value where at least one of the standardization and normalization has been performed into the trained artificial intelligence model to predict the immunogenicity of the epitope. The biological process may comprise an antigen processing stage, an antigen presentation stage, an immunogenicity stage, and a tumor microenvironment, wherein the factors involved in the tumor microenvironment may be at least one of inflammatory response, B-cell linear epitope, and B-cell conformational epitope.

Here, the device for predicting the immunogenic epitopes using an artificial intelligence model is described as comprising an input/output device 510, a storage device 520, and a computing device 530. However, a plurality of configurations may be integrated into one configuration, or a single configuration may be divided inti a plurality of configurations. In addition, a device for predicting immunogenic epitopes using an artificial intelligence model may further include a communication device, etc.

The following shows an example of predicting the immunogenic epitopes according to the method described above.

In order to learn and evaluate the immunogenicity of epitopes, data on immunogenic epitopes and non-immunogenic epitopes for neoepitopes that occur in tumors were collected from public databases and/or papers, etc.

[Table 1] below shows the number of training data sets (data sets for training) and evaluation data sets (data sets for evaluation).

[Table 1]

| Category | CD8+ T-cell immunogenic epitopes (positive) | CD8+ T-cell non-immunogenic epitopes (negative) |
|---|---|---|
| Training data sets | 315 | 4067 |
| Evaluation data sets | 79 | 3125 |

In this Example, as shown in [Table 1], a total of 315 immunogenic epitopes (positive) and 4067 non-immunogenic epitopes (negative) (i.e., epitopes and MHC I alleles binding to the epitopes) collected from dbPepNeo, NEPdb, PRIME data, and INeo-Epp data were used as training data sets, and 79 immunogenic epitopes (positive) and 3125 non-immunogenic epitopes (negative) collected from McPAS-TCR, VDJdb, IEDB t-cell db, and TESLA consortium data were used as an evaluation data sets (independent sets). To predict the immunogenicity of epitopes, each biological process and epitope properties were calculated by the programs and methods presented in [Table 2].

[Table 2]

| Biological process/ Epitope properties | Program/Algorithm/ Tool | References |
|---|---|---|
| Functional impact by the mutation | PROVEAN | PMID: 23056405 |
| Phosphorylation (phosphorylated MHC class I ligands) | NetMHCphosPan | 10.1016/j.immuno.20 21.100005 |
| Hydrophobicity | ProtFP | PMID: 24059694 |
| Similarity to known epitopes | blastP | - |
| Dissimilarity to self (human reference)-proteome | "pairwise2" module in Python Bio package | - |
| Peptide stability | ProtParam | PMID: 2075190 |
| Proteasomal cleavage | NetCTLpan | PMID: 20379710 |
| TAP transporter efficiency | NetCTLpan | PMID: 20379710 |
| Antigen processing (Proteasomal cleavage + TAP transporter efficiency) | MHCflurry (antigen processing score) | PMID: 32711842 |
| MHC I binding affinity | NetMHCpan | PMID: 32406916 |
| | MHCflurry (antigen presentation score) | PMID: 32711842 |
| pMHC stability | NetMHCstabpan | PMID: 27402703 |
| Immunogenicity (T-cell interaction) | IEDB tools | PMID: 24204222 |
| | PRIME | PMID: 33665637 |
| Inflammatory response | PIP-EL | PMID: 30108593 |
| B-cell linear epitope | Bepipred | PMID: 16635264 |
| B-cell conformational epitope | Discotope | PMID: 17001032 |

The average binding affinity to MHC I alleles, cross-reactivity to MHC I alleles, average binding affinity to MHC II alleles, and cross-reactivity to MHC II alleles proposed in the present disclosure were calculated as described above. Here, the binding affinity to MHC I alleles was calculated using NetMHCpan, and the binding affinity to MHC II alleles was calculated using the IEDB recommendation program

(http://tools.iedb.org/mhcii/). Since 3D protein structure information was required for B-cell conformational epitopes, the 3D protein structure was identified by comparing the source protein of the epitope with the human protein sequences in the AlphaFold Protein Structure database using blastP. Then, the modeller program was used to generate 3D protein structure information containing the corresponding epitope, followed by Discotope calculations. For the calculated biological process and epitope properties, the discriminatory ability to distinguish between immunogenic epitopes and non-immunogenic epitopes was confirmed based on AUC, prAUC, and P-value (Wilcoxon rank-sum test, $P < 0.05$). All biological process and epitope properties, except for NetCTLpan (TAP efficiency), statistically significantly discriminated immunogenic/non-immunogenic epitopes.

[Table 3]

| Factors/Programs | AUC | prAUC | P-value |
|---|---|---|---|
| Similarity to known epitopes | 0.561 | 0.143 | 2.91.E-04 |
| Dissimilarity to self (human reference)-proteome | 0.459 | 0.067 | 1.53.E-02 |
| Hydrophobicity (ProtFP) | 0.550 | 0.078 | 3.03.E-03 |
| NetCTLpan (TAP efficiency) | 0.526 | 0.079 | 0.1228 |
| NetCTLpan (Cleavage) | 0.535 | 0.083 | 3.68.E-02 |
| NetCTLpan (Comb) | 0.562 | 0.126 | 2.55.E-04 |
| NetMHCpan (%Rank_EL) | 0.652 | 0.168 | 1.90.E-19 |
| NetMHCpan (%Rank_BA) | 0.658 | 0.149 | 9.41.E-21 |
| MHCflurry (processing score) | 0.650 | 0.146 | 5.56.E-19 |
| MHCflurry (presentation score) | 0.673 | 0.177 | 9.75.E-25 |
| NetMHCStabPan (%Rank_Stab) | 0.584 | 0.123 | 5.40.E-07 |
| PRIME (%Rank) | 0.679 | 0.163 | 2.20.E-26 |
| DeepHLApan (immunogenic score) | 0.553 | 0.087 | 1.86.E-03 |
| NetMHCphosPan (Rnk_EL) | 0.626 | 0.142 | 9.12.E-14 |
| PIP-EL (inflammatory response) | 0.461 | 0.066 | 2.07.E-02 |
| PROVEAN (functional impact by the mutation | 0.432 | 0.060 | 5.64.E-05 |
| Avg. binding affinity to MHC I alleles | 0.561 | 0.091 | 3.27.E-04 |
| Cross-reactivity to MHC I alleles | 0.536 | 0.087 | 3.19.E-02 |
| B-cell linear epitope (BcellPred) | 0.451 | 0.063 | 4.01.E-03 |
| Peptide stability | 0.456 | 0.066 | 8.78.E-03 |
| Avg. binding affinity to MHC II alleles | 0.591 | 0.106 | 7.67.E-08 |
| Cross-reactivity to MHC II alleles | 0.597 | 0.106 | 6.10.E-10 |
| B-cell conformational epitope (Discotope) | 0.446 | 0.062 | 1.37.E-03 |

A total of 22 statistically significant factors for the epitopes in the training data set/evaluation data set were matrixed in two dimensions (rows: epitope-HLA alleles, columns: 22 factors) to perform machine learning analysis. Here, the 22 factors are factors involved in immunogenicity-related biological processes and epitope properties, and factors or properties measured by multiple programs/algorithms/tools may be treated as multiple factors. For example, immunogenicity may be measured by PRIME and IEDB, which can be two factors. In this Example, several machine learning algorithm analyses were performed using the Weka program, and finally the final model was generated by integrating and selecting several machine learning algorithms. In this Example, the final model was selected based on the average probability of the model results by nine machine learning algorithms: LogitBoost, BayesNet, CSForest, AdaBoost, Logistic, PART, NaiveBayes, RandomForest, and SMO, and in order to compare and evaluate the degree of performance improvement of the present disclosure, published immunogenicity-related programs were performed on the same data and the performances were compared. [Table 4] below shows the results of the epitope immunogenicity prediction performance evaluation for the training data sets, and [Table 5] shows the results of the epitope immunogenicity prediction performance evaluation for the evaluation data sets.

[Table 4]

| Algorithm | Threshold | Training DB (P:315, N:4067) | | | | | |
|---|---|---|---|---|---|---|---|
| | | F-Measure | Precision | Recall | Accuracy | ROC Area (=AUC ) | PRC Area (=prAUC) |
| NetMHCpan (%Rank EL) | < 0.5 | **0.193** | **0.116** | **0.565** | **0.660** | **0.652** | **0.168** |
| MhcFlurry-2.0 (presentation_score) | > 0.5 | **0.179** | 0.103 | 0.689 | 0.546 | 0.673 | 0.177 |
| PRIME (%Rank) | < 0.5 | **0.210** | 0.131 | 0.524 | 0.716 | 0.679 | 0.163 |
| DeepHLApan (Immunogenic_score ) | > 0.5 | **0.145** | 0.083 | 0.590 | 0.499 | 0.553 | 0.087 |
| IEDB tool | > 0.0 | **0.137** | 0.078 | 0.578 | 0.478 | 0.532 | 0.080 |
| DeepImmuno | > 0.5 | 0.158 | 0.087 | 0.934 | 0.187 | 0.548 | 0.091 |
| TESLA consortium standards | - | 0.194 | 0.164 | 0.238 | 0.858 | 0.572 | 0.228 |
| The present disclosure (NeoPro-Onco) | > 0.5 | 0.301 | 0.251 | 0.378 | 0.874 | 0.732 | 0.233 |

[Table 5]

| Algorithm | Threshold | Independent test DB (P:79, N:3125) | | | | | |
|---|---|---|---|---|---|---|---|
| | | F-Measure | Precision | Recall | Accuracy | ROC Area (=AUC) | PRC Area (=prAUC) |
| NetMHCpan (%Rank_EL) | < 0.5 | 0.285 | 0.176 | 0.747 | 0.908 | 0.942 | 0.317 |
| MhcFlurry-2.0 (presentation_score) | > 0.5 | 0.289 | 0.175 | 0.835 | 0.899 | 0.949 | 0.325 |
| PRIME (%Rank) | < 0.5 | 0.323 | 0.208 | 0.722 | 0.925 | 0.919 | 0.328 |
| DeepHLApan (Immunogenic_score ) | > 0.5 | 0.059 | 0.031 | 0.468 | 0.629 | 0.538 | 0.032 |
| IEDB tool | > 0.0 | 0.061 | 0.032 | 0.633 | 0.519 | 0.598 | 0.032 |
| DeepImmuno | > 0.5 | 0.110 | 0.058 | 0.892 | 0.139 | 0.608 | 0.099 |
| TESLA consortium standards | - | 0.382 | 0.385 | 0.380 | 0.970 | 0.682 | 0.390 |
| The present disclosure (NeoPro-Onco) | > 0.5 | 0.432 | 0.358 | 0.544 | 0.965 | 0.956 | 0.434 |

In the case of the present disclosure, it is the result of performing 10-fold cross-validation, and in the case of existing published programs and algorithms, it is the result of evaluating the entire data. Referring to [Table 4], it can be confirmed that the method according to the present disclosure shows better prediction accuracy in AUC, prAUC, F-measure, etc. than the existing published program and algorithm methods. Referring to [Table 5], it can be confirmed that the method according to the present disclosure shows better prediction accuracy in AUC, prAUC, F-measure, etc. than the existing published program and algorithm methods even for the evaluation data set that was not used for learning. [Table 6] below compares the results of evaluating the epitope immunogenicity prediction performance by integrating only the factors used for CD8+ T cell immunogenicity and evaluating the epitope immunogenicity prediction performance by considering the factors newly proposed in the present disclosure (functional impact by mutation, peptide stability, average binding affinity to MHC I alleles, cross-reactivity to MHC I allele, average binding affinity to MHC II alleles, cross-reactivity to MHC II alleles, inflammatory response, B-cell linear epitope, and B-cell conformational epitope).

[Table 6]

| Algorithm | Threshold | Training DB (P:315, N:4067) | | Independent test DB (P:79, N:3125) | |
|---|---|---|---|---|---|
| | | ROC Area (=AUC) | PRC Area (=prAUC) | ROC Area (=AUC) | PRC Area (=prAUC) |
| Final model (using all factors) | > 0.5 | 0.732 | 0.233 | 0.956 | 0.434 |
| Previous model (excluding factors proposed in the present disclosure) | > 0.5 | 0.707 | 0.207 | 0.960 | 0.425 |

[Referring to Table 6, it can be seen that when the newly proposed properties of the present disclosure are considered together, higher accuracy may be obtained compared to the prAUC criterion, which is suitable for the evaluation of imbalanced data.

While the inventive concept has been particularly shown and described with reference to exemplary embodiments thereof, it will be understood by those of ordinary skill in the art that various changes in form and details may be made therein without departing from the spirit and scope of the inventive concept as defined by the following claims. It is therefore desired that the embodiments be considered in all respects as illustrative and not restrictive, reference being made to the appended claims rather than the foregoing description to indicate the scope of the disclosure.

**Claims**

1. A method for predicting the immunogenicity of an epitope, comprising:

   calculating the degree to which each of factors involved in a biological process for the epitope of a tumor cell and properties of the epitope affects the immunogenicity of the epitope;
   performing at least one of standardization and normalization on the calculated value; and
   inputting a value in which at least one of the standardization and normalization has been performed into a pre-trained artificial intelligence model to predict the immunogenicity of the epitope,
   wherein the biological process comprises an antigen processing stage, an antigen presentation stage, an immunogenicity stage, and a tumor microenvironment, and
   the factors involved in the tumor microenvironment are at least one of inflammatory response, B-cell linear epitope, and B-cell conformational epitope.

2. The method of claim 1, wherein the calculating of the degree to which each of the factors involved in the biological process for the epitope and the properties of the epitope affects the immunogenicity of the epitope comprises calculating the degree using an algorithm, a program, or a tool.

3. The method of claim 1, wherein the properties of the epitope are at least one of functional impact by mutations, phosphorylation, hydrophobicity, similarity to known epitopes, dissimilarity to self (human reference)-proteome, and peptide stability.

4. The method of claim 1, wherein the factors involved in the antigen processing stage of the biological process are at least one of proteasomal cleavage and TAP transporter efficiency.

5. The method of claim 1, wherein the factors involved in the antigen presentation stage of the biological process are at least one of MHC I binding affinity, pMHC stability, average binding affinity to MHC I alleles, cross-reactivity to MHC I alleles, average binding affinity to MHC II alleles, and cross-reactivity to MHC II alleles.

6. The method of claim 5, wherein the average binding affinity to MHC I/MHC II alleles is calculated as in [Equation 3]:

[Equation 3]

$$\sum_{i}^{n} Fi \cdot Bi$$

where n is the type/number of MHC I/MHC II alleles, F is the frequency of occurrence in all or a specific number of the corresponding MHC I/MHC II alleles, and B is the binding affinity of the epitope to the corresponding MHC I/MHC II alleles,

wherein the cross-reactivity to MHC I/MHC II alleles is a value obtained by collecting MHC I/MHC II alleles occurring in a population, calculating the binding affinity to all or a specific number of MHC I/MHC II alleles for the epitope to be calculated, and calculating a case where the binding affinity is less than a predetermined reference value to calculate how many MHC I/MHC II alleles the epitope is to react with.

7. A computer-readable recording medium recording a computer program for executing the method of any one of claims 1 to 6.

8. A device for predicting the immunogenicity of an epitope, comprising:

an input/output device receiving an epitope or outputting a prediction result of the immunogenicity of the epitope;
a storage device storing an artificial intelligence model trained to predict the immunogenicity of an epitope, based on at least some of factors involved in a biological process for an epitope of a tumor cell and properties of the epitope; and
a computing device calculating the degree to which each of the factors involved in the biological process for the epitope of the tumor cell and the properties of the epitope affects the immunogenicity of the epitope, performing at least one of standardization and normalization on the calculated value, and inputting a value where at least one of the standardization and normalization has been performed into the trained artificial intelligence model to predict the immunogenicity of the epitope,
wherein the biological process comprises an antigen processing stage, an antigen presentation stage, an immunogenicity stage, and a tumor microenvironment, and
the factors involved in the tumor microenvironment are at least one of inflammatory response, B-cell linear epitope, and B-cell conformational epitope.

**FIG. 1**

Source: Aude-Hélène Capietto et al, Curr Opin Immunol (2017) reprocessed

# FIG. 2

6. Peptide stability

5. Dissimilarity to self-proteome

4. Similarity to known epitopes

3. Hydrophobicity (Physicochemical)

2. Phosphorylation (PTM)

1. Functional impact by mutations

7-2. Antigen processing (TAP transport efficiency)

7-1. Antigen processing (Proteasomal cleavage)

13. Cross-reactivity to MHC II alleles

12. Avg. binding affinity to MHC II alleles

11. Cross-reactivity to MHC I alleles

10. Avg. binding affinity to MHC I alleles

9. pMHC stability

8. MHC class I Binding affinity

17. B-cell conformational epitope

16. B-cell linear epitope

14. Immunogenicity (TCR recognition)

15. Inflammatory response (Pro- / Anti -)

Epitope properties | Antigen processing | Antigen presentation | Immunogenicity | TME

# FIG. 3

Epitope properties

Biological processes

epitope

| Functional impact | Phospho-rylation (PTM) | Hydro-phobicity | Similarity to known epitopes | Dissimilarity to self-proteome | Peptide stability | Proteasomal cleavage | TAP transporter efficiency | MHC I binding affinity | pMHC stability | Avg. binding affinity to MHC I | Cross-reactivity to MHC I | Avg. binding affinity to MHC II | Cross-reactivity to MHC II | Immuno-genicity | Inflam-matory response | B-cell linear epitope | B-cell conform-ational epitope |

Antigen processing — Antigen presentation — Tumor microenvironment

**Standardization and/or normalization**

**Artificial intelligence model**

Predicted value of immunogenicity for epitope

EP 4 618 088 A1

# FIG. 4

Calculating degree to which each of factors involved in
biological process for epitope of tumor cell and
properties of epitope affects immunogenicity of epitope ~~ S410

Performing at least one of
standardization and normalization on calculated values ~~ S420

Inputting value in which at least one of standardization and
normalization has been performed into pre-trained
artificial intelligence model to predict immunogenicity of the epitope ~~ S430

# FIG. 5

## INTERNATIONAL SEARCH REPORT

| International application No. |
| --- |
| **PCT/KR2023/017782** |

| A. | CLASSIFICATION OF SUBJECT MATTER |
| --- | --- |

**G16B 5/00**(2019.01)i; **G16B 20/20**(2019.01)i; **G16B 40/00**(2019.01)i

According to International Patent Classification (IPC) or to both national classification and IPC

| B. | FIELDS SEARCHED |
| --- | --- |

Minimum documentation searched (classification system followed by classification symbols)

G16B 5/00(2019.01); G06F 19/16(2011.01); G16B 20/00(2019.01); G16B 30/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 면역원성 에피토프(immunogenic epitope), 예측(prediction), 항원 (antigen), 종양 미세 환경(tumor microenvironment), B-세포(B-cells)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | SCHMIDT, J. et al. Prediction of neo-epitope immunogenicity reveals TCR recognition determinants and provides insight into immunoediting. Cell Reports Medicine. 16 February 2021, vol. 2, document 100194, pp. 1-14. See entire document. | 1-8 |
| A | KIM, S. et al. Neopepsee: accurate genome-level prediction of neoantigens by harnessing sequence and amino acid immunogenicity information. Annals of Oncology. 2018, vol. 29, pp. 1030–1036. See entire document. | 1-8 |
| A | SMITH, C. C. et al. Machine-Learning Prediction of Tumor Antigen Immunogenicity in the Selection of Therapeutic Epitopes. Cancer Immunology Research. 2019, vol. 7, pp. 1591–1604. See entire document. | 1-8 |
| A | KR 10-2016-0030101 A (BIONTECH AG et al.) 16 March 2016 (2016-03-16) See entire document. | 1-8 |
| A | KR 10-2015-0021528 A (BAYER HEALTHCARE LLC) 02 March 2015 (2015-03-02) See entire document. | 1-8 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **07 February 2024** | **07 February 2024** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/017782**

| Patent document cited in search report | | | Publication date (day/month/year) | Patent family member(s) | | | Publication date (day/month/year) |
|---|---|---|---|---|---|---|---|
| KR | 10-2016-0030101 | A | 16 March 2016 | CA | 2911945 | A1 | 13 November 2014 |
| | | | | CA | 2911945 | C | 17 October 2023 |
| | | | | CN | 105451759 | A | 30 March 2016 |
| | | | | CN | 105451759 | B | 05 February 2021 |
| | | | | CN | 113219179 | A | 06 August 2021 |
| | | | | CY | 1124176 | T1 | 27 May 2022 |
| | | | | DK | 2994159 | T3 | 25 May 2021 |
| | | | | E0 | 55146 | T2 | 29 November 2021 |
| | | | | EP | 2994159 | A1 | 16 March 2016 |
| | | | | EP | 2994159 | B1 | 10 March 2021 |
| | | | | EP | 3895727 | A1 | 20 October 2021 |
| | | | | ES | 2871384 | T3 | 28 October 2021 |
| | | | | HK | 1215169 | A1 | 19 August 2016 |
| | | | | IL | 242281 | B | 30 April 2020 |
| | | | | IL | 273785 | A | 31 May 2020 |
| | | | | JP | 2016-521128 | A | 21 July 2016 |
| | | | | JP | 2020-103297 | A | 09 July 2020 |
| | | | | JP | 2021-121801 | A | 26 August 2021 |
| | | | | JP | 6710634 | B2 | 17 June 2020 |
| | | | | JP | 6882550 | B2 | 02 June 2021 |
| | | | | JP | 7405792 | B2 | 26 December 2023 |
| | | | | KR | 10-2399419 | B1 | 19 May 2022 |
| | | | | LT | 2994159 | T | 25 August 2021 |
| | | | | MX | 2015015511 | A | 09 December 2016 |
| | | | | NZ | 714059 | A | 27 August 2021 |
| | | | | P2 | 0210773 | T1 | 17 September 2021 |
| | | | | PL | 2994159 | T3 | 02 August 2021 |
| | | | | PT | 2994159 | T | 28 May 2021 |
| | | | | RS | 61868 | B1 | 30 June 2021 |
| | | | | RU | 2015-153007 | A | 16 June 2017 |
| | | | | RU | 2020-110192 | A | 04 August 2020 |
| | | | | RU | 2724370 | C2 | 23 June 2020 |
| | | | | SG | 11201508816 | RA | 27 November 2015 |
| | | | | SI | 2994159 | T1 | 30 July 2021 |
| | | | | US | 11222711 | B2 | 11 January 2022 |
| | | | | US | 2016-0125129 | A1 | 05 May 2016 |
| | | | | US | 2022-0093209 | A1 | 24 March 2022 |
| | | | | WO | 2014-180490 | A1 | 13 November 2014 |
| | | | | WO | 2014-180569 | A1 | 13 November 2014 |
| | | | | ZA | 201508048 | B | 31 May 2017 |
| KR | 10-2015-0021528 | A | 02 March 2015 | BR | 112014029235 | A2 | 29 January 2019 |
| | | | | CA | 2874542 | A1 | 28 November 2013 |
| | | | | CN | 104487979 | A | 01 April 2015 |
| | | | | EP | 2856374 | A1 | 08 April 2015 |
| | | | | EP | 2856374 | A4 | 20 April 2016 |
| | | | | IL | 235815 | A | 29 January 2015 |
| | | | | JP | 2015-526775 | A | 10 September 2015 |
| | | | | MX | 2014014199 | A | 12 February 2015 |
| | | | | NZ | 702084 | A | 30 September 2016 |
| | | | | RU | 2014-152463 | A | 20 July 2016 |

Form PCT/ISA/210 (patent family annex) (July 2022)

**INTERNATIONAL SEARCH REPORT**
Information on patent family members

International application No.

**PCT/KR2023/017782**

| Patent document cited in search report | Publication date (day/month/year) | Patent family member(s) | | Publication date (day/month/year) |
|---|---|---|---|---|
| | | SG 11201407459 | QA | 30 December 2014 |
| | | US 2015-0205911 | A1 | 23 July 2015 |
| | | WO 2013-176756 | A1 | 28 November 2013 |

Form PCT/ISA/210 (patent family annex) (July 2022)